# FASCICULE DE BREVET EUROPEEN

(11) **EP 2 987 452 B1**
(45) Date de publication et mention de la délivrance du brevet: **05.04.2023**
(21) Numéro de dépôt: 15181662.6
(22) Date de dépôt: 20.08.2015
(51) Int. Cl.: A61B 5/00

(54) **PROCÉDÉ ET SYSTÈME DE SÉLECTION AUTOMATIQUE D'EXERCICES PHYSIQUES**
VERFAHREN UND SYSTEM ZUR AUTOMATISCHEN AUSWAHL VON KÖRPERLICHEN ÜBUNGEN
METHOD AND SYSTEM FOR AUTOMATIC SELECTION OF PHYSICAL EXERCISES

(30) Priorité: 21.08.2014 CH 12572014
(43) Date de publication de la demande: 24.02.2016
(73) Titulaire: Myotest SA, 1950 Sion (CH)
(72) Inventeur: FLACTION, Patrick, 1965 Chandolin-près-Savièse (CH); GINDRE, Cyrille, 1854 Leysin (CH)
(74) Mandataire: P&TS SA (AG, Ltd.)

(56) Documents cités:
- US-A1- 2008 171 921

## Description

### Domaine technique

La présente invention concerne un procédé et un système de sélection automatique d'exercices physiques à proposer à un utilisateur, par exemple un athlète, une personne âgée ou toute autre personne.

### Etat de la technique

Un athlète désirant améliorer ses performances athlétiques, une personne souhaitant devenir plus mince ou améliorer son bien-être, ou une personne âgée désirant monter plus efficacement des escaliers sont des exemples non limitatifs de personnes nécessitant d'exécuter des exercices physiques.

Dans le contexte de cette invention le terme « exercice physique » désigne des exercices d'entraînement, des exercices de réhabilitation ou de manière générale tous les exercices physiques correspondant à toute situation de mise en mouvement d'au moins une partie du corps d'une personne. Ces exercices sont en général proposés à une personne dans un but sportif, de réhabilitation ou de santé.

Des exercices physiques sont souvent proposés par un coach ou expert, qui décide non seulement du plan d'entraînement ou réhabilitation pour la personne à entraîner ou réhabiliter (athlète, personne âgée, etc.), mais détermine aussi avec cette personne l'objectif de ce plan, par exemple améliorer la réactivité, maigrir, améliorer la coordination des jambes, etc.

En d'autres mots les exercices sont imposés par le coach à une personne à entraîner selon un modèle « top-down ».

US2007219059 décrit une méthode et un dispositif pour le monitorage et la proposition d'un plan d'exercices mis à jour en continu. Un utilisateur peut obtenir un plan d'entrainement à l'aide d'une base de données d'exercices, servant de catalogue d'exercices à un coach qui, à distance, peut proposer un plan d'entraînement personnalisé. Dans ce cas aussi c'est le coach qui décide le plan d'entraînement pour l'utilisateur.

Le document EP2263534, déposé par la demanderesse, décrit une méthode pour l'optimisation de plans d'entraînement dans laquelle des accélérations de l'utilisateur sont mesurées lors d'un test initial par un dispositif mobile, afin de calculer des paramètres représentant les caractéristiques musculaires de l'utilisateur. Des exercices adaptés à ces caractéristiques musculaires sont déterminés et proposés à l'utilisateur. Ces exercices peuvent être sélectionnés automatiquement afin d'améliorer les paramètres musculaires les plus faibles.

Cependant cette sélection vise uniquement à améliorer les paramètres musculaires les plus faibles de l'utilisateur, sans tenir compte des objectifs de l'utilisateur. Par exemple, un sprinteur pourra se contenter d'une endurance limitée, et aura besoin d'améliorer son explosivité et sa puissance musculaire même si ces qualités physiques sont déjà très bonnes. A l'inverse, un utilisateur dont le seul objectif est de perdre du poids ne voudra peut-être pas augmenter sa masse et sa puissance musculaire même ces paramètres sont insuffisants.

Un bon coach tient généralement compte du feeling de l'utilisateur pendant l'exécution des exercices ; il ne se fie donc pas uniquement au bilan physique de l'utilisateur. A l'inverse, les systèmes de recommandation automatique d'exercices connus dans l'état de la technique ne tiennent pas compte de ce feeling. Même si les exercices proposés sont *théoriquement* adaptés à améliorer un faible paramètre musculaire de l'utilisateur, il n'y a aucune garantie que l'utilisateur qui exécute *pratiquement* ces exercices soit à l'aise pendant leur exécution. L'utilisateur n'a donc aucun moyen d'indiquer s'il se sent bien durant l'exécution d'un exercice physique.

Dans le contexte de cette invention, l'expression « sensation de l'utilisateur pendant l'exécution d'un exercice » indique les sentiments, les émotions, et en général ce que l'utilisateur ressent lors de l'exécution de cet exercice. Par exemple un utilisateur peut être à l'aise, c'est-à-dire se sentir bien, en courant de façon asymétrique, par exemple en utilisant plus la jambe droite que la jambe gauche, même si ce type de course asymétrique ne lui sera probablement jamais conseillée par un coach ou un système de sélection automatique d'exercices d'entraînement. Un utilisateur peut aussi se sentir bien en courant d'abord lentement puis en accélérant à la fin, plutôt que courir d'abord rapidement avant de décélérer. Enfin certains utilisateurs sont à l'aise en répétant le même exercice plusieurs fois, alors que d'autres préfèrent changer souvent le type d'exercice exécuté.

GB2447915 décrit un dispositif comprenant une base de données pour la création d'un plan d'entraînement personnalisé par rapport au sport et aux caractéristiques de l'utilisateur. Le dispositif comprend un dispositif multimédia et une base de données qui, en se basant seulement sur les réponses à un questionnaire de demandes, permettent à l'utilisateur ou à son coach de créer un plan d'entrainement. Les exercices sont donc proposés sans prendre en considération des paramètres musculaires mesurés.

US20080171921 concerne un système de détection, de suivi et de compte rendu qui permet à un utilisateur d'améliorer son style de vie. Des données indicatrices de paramètres physiologiques de l'utilisateur peuvent être données par un détecteur qui peut comprendre un accéléromètre, ou bien elles peuvent être entrées directement par l'utilisateur sur une page web. Quand un utilisateur a accès pour la première fois à ce système, il doit compléter un sondage détaillé. L'unité centrale suggère à l'utilisateur une routine de santé quotidienne sur la base de ce sondage. Chaque utilisateur a accès à cette page web (« Health manager ») comprenant un indice de santé (« Health Index »), qui donne un feedback aux utilisateurs sur leur performance dans le suivi de la routine de santé quotidienne. Ce feedback est basé sur les paramètres mesurés par le détecteur et/ou sur des données directement introduites par l'utilisateur. Cet index comprend six catégories (« Nutrition, Activity Level, Mind Centering, Sleep, Daily Activities, How you feel ») visibles sous la forme d'histogrammes. La catégorie « How you feel » indique comment se sent l'utilisateur un jour particulier et est basée sur une information subjective directement introduite par l'utilisateur.

### Bref résumé de l'invention

Un but de la présente invention est de proposer un procédé et un système de sélection automatique d'exercices physiques plus personnalisés, et qui tiennent compte des objectifs, du niveau et des sensations des utilisateurs pendant l'exécution des exercices physiques.

Un autre but est de proposer un procédé et un système de sélection automatique d'exercices physiques qui permet à l'utilisateur qui exécute ces exercices d'être à l'aise et/ou de progresser.

Un autre but de la présente invention est de proposer un procédé et un système de sélection automatique d'exercices qui ne reposent pas sur le modèle « top-down » discuté.

Un autre but de la présente invention est de proposer un procédé et un système de sélection automatique d'exercices améliorés par rapport aux procédés et système connus.

Selon l'invention, ces buts sont atteints notamment au moyen d'un procédé de sélection automatique d'exercices physiques à proposer à un utilisateur, cet utilisateur portant un capteur de mouvement, par exemple un accéléromètre, un gyroscope ou autre,
le procédé comprenant les étapes suivantes :
- classification d'une collection prédéfinie d'exercices physiques dans une base de données en fonction à la fois d'objectifs utilisateurs, de qualités physiques que ces exercices physiques permettent de renforcer, et pour chaque qualité physique de niveaux pour lesquels ces exercices physiques sont appropriés ;
- introduction par l'utilisateur de données personnelles comprenant au moins un objectif utilisateur ;
- test à l'aide dudit capteur de mouvement du niveau de l'utilisateur pour différentes qualités physiques;
- sélection automatique d'au moins un exercice physique dans cette base des données sur la base de l'objectif utilisateur introduit par l'utilisateur, d'une ou plusieurs qualités physiques à renforcer et/ou activer déterminées en fonction du niveau de l'utilisateur pour cette qualité, et d'au moins un feedback de l'utilisateur, ce feedback indiquant la sensation de l'utilisateur pendant l'exécution d'un exercice physique.

Dans le contexte de cette invention l'expression « objectif utilisateur » indique l'objectif de l'utilisateur pour lequel il va exécuter des exercices physiques. Un objectif utilisateur peut être très général (par exemple « perdre du poids »), ou bien correspondre à un sport particulier (par exemple « entraînement pour le marathon »).

Les objectifs utilisateurs peuvent donc inclure un sport pour lequel l'utilisateur souhaite s'entraîner.

Les objectifs utilisateurs peuvent également inclure des préférences de l'utilisateur (par exemple « je n'aime pas faire des exercices répétitifs »), et/ou les qualités physiques qu'il préfère ou aime améliorer.

Dans une variante le feedback inclue une commande haptique ou vocale introduite volontairement par l'utilisateur en utilisant le capteur de mouvement pour entrer cette commande pendant l'exécution d'un exercice physique afin d'indiquer une sensation.

Cette solution permet notamment de proposer des exercices physiques plus personnalisés, en tenant notamment compte des objectifs utilisateurs formulés par l'utilisateur lors de l'introduction de données personnelles initiales.

Cette solution présente aussi l'avantage par rapport à l'art antérieur d'exploiter le feedback de l'utilisateur, en reposant sur un modèle « bottom-up » : en effet le système selon l'invention tient en considération la réaction non seulement physique, mais aussi émotionnelle de l'utilisateur exécutant des exercices, qui est utilisée pour choisir les exercices à lui proposer. En d'autres mots la sélection des exercices, au lieu d'être imposée par le haut (un coach électronique), tient compte des aspirations du bas (l'utilisateur) en considérant la sensation de l'utilisateur lors de l'exécution de ces exercices.

Le système selon l'invention permet donc d'arriver à l'objectif de l'entraînement ou réhabilitation choisi par l'utilisateur grâce à une sélection d'exercices prenant en compte le ressenti de l'utilisateur, même si ces exercices ne sont pas les plus efficaces pour arriver à cet objectif d'un point de vue théorique.

Dans une variante le feedback inclue une signature du mouvement de l'utilisateur déterminée automatiquement sur la base de données d'accélérations captées par l'accéléromètre. En d'autres mots la sélection automatique des exercices peut être réalisée en fonction de la signature du mouvement de l'utilisateur pendant l'exécution d'un exercice physique.

La signature du mouvement de l'utilisateur comprend l'évolution temporelle d'au moins un des paramètres suivants et/ou une combinaison d'au moins deux des paramètres suivants : force, puissance, vitesse, stabilité, temps de réaction, temps de vol, temps de contact, raideur, réactivité, asymétrie, inclinaison, fatigue, risque de blessure, efficience gestuelle (motrice et/ou énergétique). Ces paramètres peuvent être simples, c'est-à-dire directement mesurés (par exemple le temps de contact), ou bien complexes, car provenant de l'intégration ou en général des calculs des paramètres simples (par exemple la fatigue). Dans une autre variante la signature du mouvement est lue, c'est-à-dire elle est comparée à une courbe temporelle attendue en fonction de l'exercice exécuté.

Les qualités physiques comprennent au moins deux des qualités physiques suivantes : « core stability », endurance, flexibilité, puissance, agilité, perception. La qualité physique « core stability » comprend le gainage et l'équilibre. La qualité physique « puissance » peut comprendre la puissance de la partie basse du corps de l'utilisateur (pax exemple des jambes) et/ou la puissance de sa partie haute (par exemple les bras et/ou le tronc). La qualité physique « puissance » peut comprendre la force et la vitesse. La qualité physique « agilité » peut comprendre l'équilibre, la réaction et/ou le rythme. Dans une autre variante les qualités physiques comprennent également la perception, c'est-à-dire la réaction de l'utilisateur à tout type de signal, par exemple un signal auditif et/ou visuel et/ou la capacité de discrimination visuelle. Dans une variante, la qualité physique « agilité » comprend également la perception.

Les niveaux sont au moins deux, et sont indicatifs de façon progressive des qualités physiques de l'utilisateur et/ou de la difficulté de chaque exercice physique.

Dans une variante le procédé selon l'invention comprend les étapes suivantes :
- mesure du niveau de l'utilisateur pour au moins un paramètre physique au cours de l'exécution des exercices physiques sélectionnés par le système selon l'invention, et
- ultérieure sélection de nouveaux exercices physiques en fonction de ce niveau, des objectifs de l'utilisateur et du feeling de l'utilisateur. Dans une variante cette ultérieure sélection dépend également de la signature du mouvement de l'utilisateur.

Dans une variante, les données personnelles comprenant au moins une des données suivantes : âge, poids, taille, sexe de l'utilisateur. Dans une autre variante ces données comprennent également une pathologie, c'est-à-dire une éventuelle maladie et/ou problème physique de l'utilisateur et/ou son stade de récupération, ce qui est utile notamment dans un contexte de rééducation de l'utilisateur pendant et/ou après sa maladie.

Avantageusement le test initial proposé à l'utilisateur peut évoluer et se modifier automatiquement en fonction des différentes qualités physiques mesurées pendant l'exécution des exercices physiques.

Dans une variante préférentielle, un ou plusieurs exercices physiques peuvent constituer une unité d'entraînement, qui a une durée de quelques minutes, par exemple de 10 minutes. Dans le contexte de cette invention, le mot « séance » indique alors une unité d'entraînement, ou un assemblage de plusieurs unités d'entraînement. Une séance peut donc avoir une durée totale d'un nombre entier de semaines, par exemple de 3 semaines. Dans une variante des exercices-repère sont placés dans les séances de façon à faire évoluer l'entraînement selon le niveau atteint au temps t+1 comparativement au niveau atteint au temps t. Dans une autre variante chaque séance est suivie par un test d'évaluation.

Selon l'invention, ces buts sont atteints aussi au moyen d'un système de sélection automatique d'exercices physiques à proposer à un utilisateur, comprenant :
- un capteur de mouvement arrangé pour être porté par l'utilisateur,
- une base de données dans laquelle une collection prédéfinie d'exercices physiques est classifiée en fonction à la fois d'objectifs utilisateurs, de qualités physiques que ces exercices physiques permettent de renforcer et/ou activer, et pour chaque qualité physique de niveaux d'utilisateurs pour lesquels ces exercices physiques sont appropriés,
- le système étant configuré pour permettre à l'utilisateur d'introduire de données personnelles comprenant au moins un objectif utilisateur, et de sélectionner automatiquement au moins un exercice physique dans la base des données sur la base de l'objectif utilisateur introduit par l'utilisateur, d'une ou plusieurs qualités physiques à renforcer et/ou activer déterminées en fonction du niveau de l'utilisateur pour cette qualité, et d'au moins un feedback de l'utilisateur, ce feedback indiquant la sensation de l'utilisateur pendant l'exécution d'un exercice physique.

Dans une variante préférentielle l'utilisateur porte également un dispositif mobile.

Dans une variante préférentielle le capteur de mouvement est porté près du centre de masse de l'utilisateur. Dans une variante préférentielle le capteur de mouvement est un accéléromètre, par exemple un accéléromètre triaxial. Dans une autre variante, alternative ou complémentaire à la précédente, le capteur de mouvement est un gyroscope.

Dans une autre variante, la relation entre le dispositif mobile et l'accéléromètre est du type master-slave.

### Brève description des figures

Des exemples de mise en oeuvre de l'invention sont indiqués dans la description illustrée par les figures annexées dans lesquelles :
La figure 1 illustre un utilisateur portant un dispositif mobile et un accéléromètre du système de sélection automatique d'exercices selon un mode de réalisation de l'invention.
La figure 2 illustre un mode de réalisation du dispositif mobile et de l'accéléromètre du système de sélection automatique d'exercices de la figure 1.
La figure 3A illustre un mode de réalisation des niveaux du procédé de sélection automatique d'exercices de l'invention.
La figure 3B illustre un mode de réalisation de la grille à deux dimensions (niveaux - qualités physiques) du procédé de sélection automatique d'exercices de l'invention.
La figure 3C illustre un mode de réalisation d'une espace à trois dimensions (niveaux - qualités physiques - exercices) du procédé de sélection automatique d'exercices de l'invention.
La figure 4 illustre un mode de réalisation de la correction des niveaux de chaque qualité physique après le test initial du procédé de sélection automatique d'exercices de l'invention.

### Exemple(s) de mode(s) de réalisation de l'invention

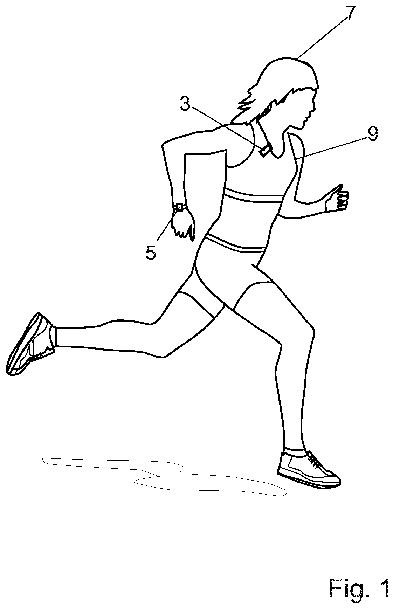

La présente invention concerne un procédé de sélection automatique d'exercices physiques à proposer à un utilisateur. Comme illustré sur la figure 1, cet utilisateur 7 porte un dispositif mobile 5, dans l'exemple illustré une montre, et un capteur de mouvement, par exemple un accéléromètre 3 configuré pour communiquer avec le dispositif mobile 5. Dans la description suivante fournie à titre d"exemple, on fera référence, par simplicité, à un accéléromètre comme capteur de mouvement. Il faut toutefois comprendre que l'invention n'est pas limitée à un tel capteur, mais inclut également d'autres capteurs de mouvement, par exemple un gyroscope, etc. Dans une variante préférentielle la communication entre le dispositif mobile 5 et l'accéléromètre 3 est sans fils.

Le dispositif mobile 5 n'est pas nécessairement une montre mais peut être, par exemple et de façon non limitative, un lecteur de musique portable, un smartphone, des lunettes, un bijou, etc. ou tout dispositif qui peut être porté par un utilisateur. Le dispositif mobile 5 et l'accéléromètre 3 peuvent aussi être intégrés en un seul objet.

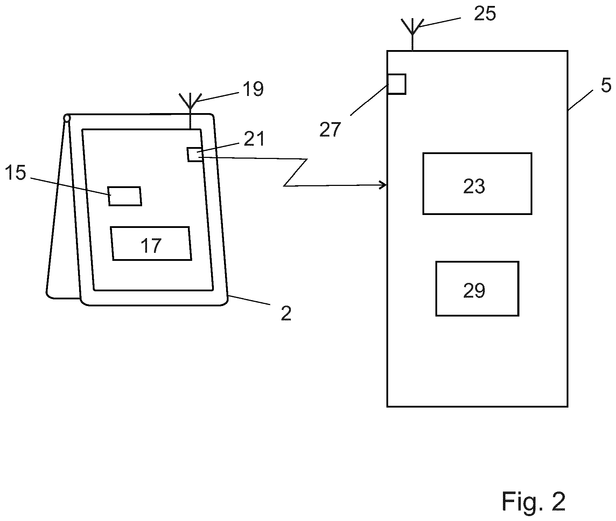

Avantageusement le dispositif mobile 5 comprend un processeur 23, visible sur la figure 2, configuré pour élaborer les données d'accélération qui lui sont transmises par l'accéléromètre 3, une mémoire 29 configurée pour stocker des données ou les résultats de l'élaboration du processeur 23, un récepteur 25 et un émetteur 27 pour communiquer avec l'accéléromètre.

L'accéléromètre 3 est configuré pour être lié de façon amovible, par exemple avec des clips 2, une ceinture ventrale, un bracelet, etc. au vêtement 9 de l'utilisateur 7 ou directement à l'utilisateur 7. Dans une autre variante l'accéléromètre est dans une ou relié à une chaussure de l'utilisateur 7.

Avantageusement l'accéléromètre 3 comprend des moyens de mesure de l'accélération 15 (capteur d'accélération, par exemple triaxial), visibles sur la figure 2, une mémoire 15, un récepteur 19 et un émetteur 21 pour communiquer avec le dispositif mobile 5. L'accéléromètre peut comprendre également un processeur non illustré.

Avantageusement le dispositif mobile 5 agit en tant que maître par rapport à l'accéléromètre 3 (esclave), car il peut demander des données d'accélération à l'accéléromètre 3 seulement lors de certains événements, comme décrit dans le document WO2012171967 déposé par la demanderesse et qui est incorporé ici par référence.

Le procédé selon l'invention comprend les étapes suivantes :
- classification d'une collection prédéfinie d'exercices 300 dans une base de données en fonction à la fois d'objectifs utilisateurs sélectionnés préalablement par l'utilisateur 7, de qualité physiques 200 que chaque exercice 300 permet de renforcer et/ou activer, et de niveaux 100 de l'utilisateur 7 et/ou de l'exercice 300 pour chaque qualité physique 200 ;
- sélection automatique par le dispositif mobile 5 et/ou par l'accéléromètre 3 d'au moins un exercice dans la base de données.

Dans une autre variante la sélection est réalisée par tout autre moyen de calcul tel qu'un PC, un laptop, une tablette, etc. qui n'est pas porté par l'utilisateur, et qui est relié au dispositif mobil 5 et/ou à l'accéléromètre 3 par une connexion sans ou avec fils.

Avantageusement cette sélection automatique est réalisée sur la base de l'objectif utilisateur introduit par l'utilisateur 7, d'une ou plusieurs qualités physiques 200 à renforcer et/ou activer déterminées en fonction du niveau 100 de l'utilisateur pour cette qualité tel que mesuré à l'aide de l'accéléromètre 3, et d'au moins un feedback de l'utilisateur.

Les objectifs utilisateurs sélectionnés par l'utilisateur peuvent être choisis dans une liste d'objectifs utilisateurs prédéfinis. Comme discuté, il peut s'agit d'objectifs généraux, par exemple « remise en forme », « perte de poids », ou des objectifs liés à un sport et/ou à une pathologie en rééducation, par exemple « entraînement pour le ski », « préparation d'un marathon », « entraînement pour rééduquer le genou gauche », etc.

Avantageusement le feedback de l'utilisateur 7 indique la sensation de l'utilisateur 7 pendant l'exécution d'un exercice. Comme discuté, dans le contexte de cette invention l'expression « sensation de l'utilisateur pendant l'exécution d'un exercice » indique le « feeling », les sentiments, les émotions, et en général ce que l'utilisateur 7 éprouve lors de l'exécution de cet exercice.

Avantageusement la classification d'une collection prédéfinie d'exercices dans une base de données est réalisée au préalable par un coach ou expert sur la base de ses connaissances et de son savoir-faire.

Les exercices 300 peuvent être classifiés en fonction :
1. des niveaux 100 que l'exercice requiert pour chaque qualité physique;
2. des qualités physiques 200 que les exercices permettent de renforcer et/ou activer;
3. des objectifs utilisateurs sélectionnés par l'utilisateur 7.

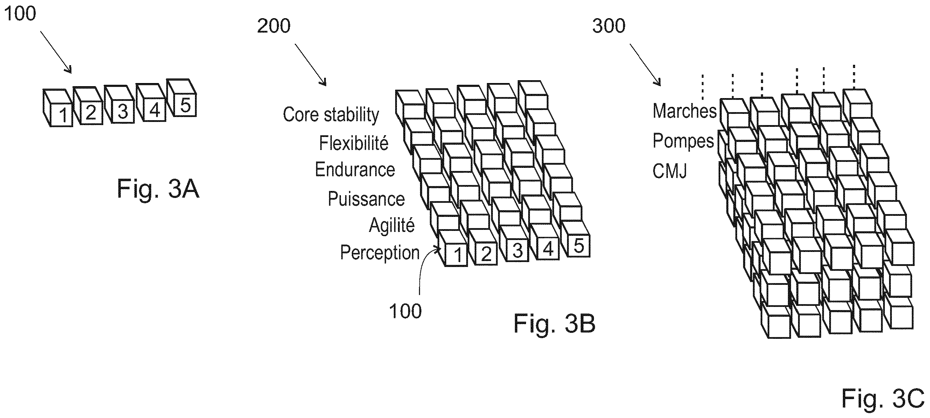

Le nombre de niveaux 100 est un nombre entier, par exemple 5 comme illustré sur la figure 3A, le niveau 1 étant par exemple le niveau le plus facile, le niveau 5 étant par exemple le niveau le plus difficile, les niveaux intermédiaires 2 à 4 représentant de façon progressive une augmentation de difficulté.

Ces cinq niveaux sont utilisés pour classifier les différents utilisateurs en fonction des résultats qu'ils obtiennent pour chaque qualité physique lors d'un test initial. Ainsi une personne débutant la course à pied parviendra au niveau 1 pour la qualité physique « endurance » ; un marathonien d'élite atteindra le niveau 5.

Les différents exercices 300 stockés dans la base de données peuvent également être associés à des niveaux 100. Ainsi un tour de piste de 400 mètres à un rythme léger sera adapté à des utilisateurs au niveau 1 ou 2 de la qualité physique endurance ; un entraînement rapide sur 20 kilomètres convient à des utilisateurs au niveau 4 ou 5 de cette qualité physique.

Un même exercice 300 peut être associé à différentes qualités physiques 200 et à différents niveaux 100. Par exemple, une course à la montée sera aussi bien utile pour améliorer l'endurance que la puissance des membres inférieurs.

Dans un mode de réalisation préférentiel il est possible de passer d'un niveau à celui qui le précède ou le suit par incréments de 1 (par exemple il est possible de passer du niveau 2 au niveau 1 ou 3, mais pas directement au niveau 4). Dans une autre variante préférentielle, les incréments peuvent être plus petits, par exemple de 0.1, ce qui permet d'avoir une plus grande résolution pour classer des exercices. Dans une autre variante il est possible de réaliser des sauts d'un niveau vers n'importe quel autre niveau.

Dans une variante préférentielle les qualités physiques 200 que les exercices permettent de renforcer et/ou activer sont six :
1. « Core stability », qui comprend la notion de gainage et d'équilibre,
2. Flexibilité,
3. Endurance,
4. Puissance, qui comprend la puissance bas (c'est-à-dire de la partie basse de l'utilisateur, par exemple de ses jambes) et/ou la puissance haut (c'est-à-dire de la partie haute de l'utilisateur, par exemple de ses bras),
5. Agilité, qui comprend par exemple la coordination,
6. Perception, qui comprend toute réaction physique à un signal.

En utilisant les niveaux 100 et les qualités physiques 200, il est donc possible de classer les exercices dans un espace à deux dimensions (niveau × qualité physique), dont un mode de réalisation est illustré sur la figure 3B. Pour chaque exercice cette classification permet d'attribuer au moins un niveau 100 pour chaque qualité physique 200 que l'exercice permet d'entraîner.

Avantageusement cet espace à deux dimensions peut être représenté sous la forme d'une grille ou tableau, ayant sur l'axe horizontal les niveaux 100 et sur l'axe vertical les qualités physiques 200.

Chaque exercice est classifié dans cette grille en spécifiant le pourcentage de chaque qualité physique qui est requis pour un niveau donné. La somme des pourcentages de la grille doit être 100%.

Par exemple ci-dessous la grille en deux dimensions pour l'exercice CMJ bras libre :

| | | Niveau 1 | Niveau 2 | Niveau 3 | Niveau 4 | Niveau 5 |
|---|---|---|---|---|---|---|
| Core stability | | | | | | |
| Flexibilité | | | | | | |
| Endurance | | | | | | |
| Puissance | bas | | | 90% | | |
| | haut | | 10% | | | |
| Agilité | | | | | | |
| Perception | | | | | | |

Cette grille indique que l'exercice CMJ bras libre permet d'exercer les qualités physiques « puissance bas » et « puissance haut ». Cette grille indique notamment que cet exercice est approprié pour un utilisateur qui a un niveau moyen (niveaux 3) de la qualité physique « puissance bas » et un niveau un peu inférieur à la moyenne (niveau 2) pour la « puissance haut ». Les pourcentages indiquent que cet exercice contribue à 90% à améliorer et/ou solliciter la puissance bas, et pour 10% à améliorer la puissance haut.

Un même exercice peut être recommandé à plusieurs types d'utilisateurs ayant des niveaux différents pour une ou plusieurs qualités physiques. Par exemple, l'exercice ci-dessus pourrait aussi être adapté à un utilisateur ayant un niveau 2 pour la « puissance bas » et un niveau 1 pour la puissance « puissance haut ».

Ce classement de chaque exercice dans cette grille à deux dimensions correspond à l'ajout d'une troisième dimension, comme visible sur la figure 3C. En effet chaque exercice 300 (marche, pompes, CMJ, etc.) correspond à une grille bidimensionnelle telle que celle illustrée ci-dessus.

Le marquage ou classification de chaque exercice 300 dans l'espace en deux dimensions de la figure 3B dépend également :
- du type d'exercice ;
- d'une consigne donnée avec l'exercice, par exemple du nombre de fois que l'exercice doit être répété, de la charge qui doit être déplacée, de la durée du repos entre chaque répétition, etc. Par exemple, un exercice de type CMJ peut être classé différemment si la consigne est de le répéter une fois ou plusieurs fois, ou de l'effectuer avec une charge supplémentaire sur le corps.

Dans une variante préférentielle, chaque objectif utilisateur peut également être classé dans une grille bidimensionnelle telle que celle représentée sur la figure 3B.

Par exemple ci-dessous la grille en deux dimensions pour un objectif utilisateur « anti-âge » :

| | | Niveau 1 | Niveau 2 | Niveau 3 | Niveau 4 | Niveau 5 |
|---|---|---|---|---|---|---|
| Core stability | | | 10% | | | |
| Flexibilité | | | 10% | | | |
| Endurance | | | 20% | | | |
| Puissance | bas | | 20% | | | |
| | haut | | 10% | | | |
| Agilité | | | 20% | | | |
| Perception | | | 10% | | | |

Cette grille indique que cet objectif contribue à 20% à améliorer l'agilité, à 20% à améliorer l'endurance, à 10% à améliorer la flexibilité, etc. Cette grille indique que pour atteindre l'objectif, il convient de réserver 20% des unités d'entraînement à l'agilité, 20% à l'endurance, 10% à la flexibilité, etc.

Les règles suivantes peuvent être utilisées pour modifier cette classification :
- plus l'âge de l'utilisateur 7 augmente et plus les niveaux des qualités physiques requises diminuent;
- si le sexe est féminin, le niveau de gainage, puissance (bas et haut) et endurance requis diminue ;
- si l'IMC est plus élevé par rapport à l'IMC moyen d'une personne ayant le même sexe et le même âge que l'utilisateur 7, les niveaux théoriques des qualités physiques diminuent.

Le procédé selon l'invention permet avantageusement de fournir des règles qui permettent de relier l'utilisateur 7, notamment ses caractéristiques, les exercices 300 et les objectifs utilisateurs.

Avant de commencer à faire des exercices, l'utilisateur, introduit des données personnelles dans le dispositif mobile 5, dans l'accéléromètre 3 ou dans tout autre dispositif connecté, sans ou avec fils, au dispositif mobile 5 ou à l'accéléromètre 3, par exemple un ordinateur, un PDA, un laptop, etc.

Dans une variante préférentielle ces données comprennent le nom de l'utilisateur 7, ainsi que :
- Son âge
- Son sexe
- Son poids et sa taille (nécessaires pour le calcul de l'IMC - Indice Masse Corporelle)
- Son objectif initial (par exemple remise en forme, bien-être, entraînement sportif, etc.). L'objectif introduit peut indiquer l'entraînement à un sport particulier choisi dans une liste de sports. Dans une variante ces données comprennent également une pathologie, c'est-à-dire une éventuelle maladie et/ou problème physique de l'utilisateur et/ou son stade de récupération, ce qui est utile notamment dans un contexte de rééducation de l'utilisateur pendant et/ou après sa maladie.

Le système propose ensuite n ou plusieurs exercices *initiaux* à l'utilisateur.

Pendant l'exécution de ces exercices initiaux, l'accéléromètre 3 mesure des accélérations qui sont transmises au dispositif mobile 5. Les tests proposés (exercices repère) peuvent dépendre des données personnelles introduites par l'utilisateur. Par exemple, un utilisateur âgé et corpulent ne se verra pas proposer les mêmes tests initiaux (exercices repère) de mesure d'endurance qu'un utilisateur jeune avec un objectif d'entraînement pour la course à pied.

Dans une variante préférentielle, ces exercices initiaux sont sélectionnés sur la base de l'âge, du sexe, de l'IMC de l'utilisateur, de sa pathologie et de son objectif initial.

Les tests physiques initiaux sont de préférence constitués par une série d'exercices assez courts, comprenant par exemple une série de sauts, une course brève, etc.

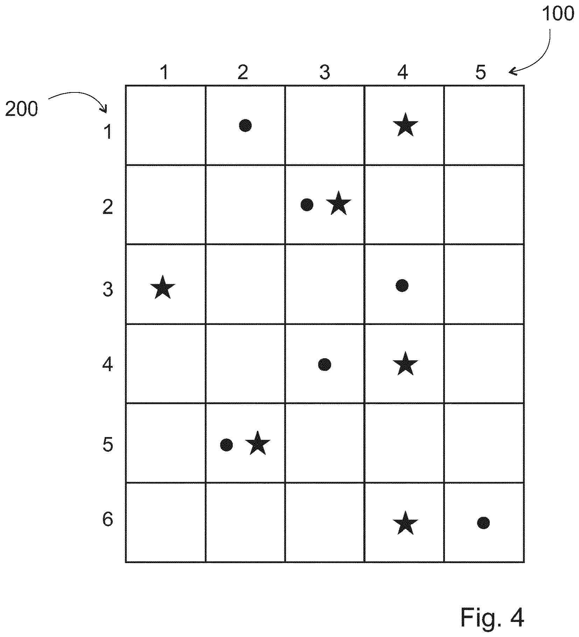

Les données personnelles introduites par l'utilisateur 7 permettent de déterminer le niveau théorique de l'utilisateur 7 pour chaque qualité physique prédéterminée. Cette classification peut être effectuée par le dispositif mobile 5 (ou par un dispositif connecté au dispositif mobile 5) et permet de déterminer, pour chaque qualité physique, un niveau théorique allant par exemple de 1 (débutant) à 5 (expert). En d'autres mots chaque utilisateur 7 est classé dans une grille bidimensionnelle similaire à celle de la figure 3B, qui avait été utilisée pour classer des exercices et qui est maintenant utilisée pour classer l'utilisateur 7. Ce niveau théorique est représenté par des points ronds sur la figure 4.

Les mesures effectuées lors des tests physiques initiaux permettent quant à elles de calculer les qualités physiques de l'utilisateur 7 (représentées par des étoiles sur la figure 4) et de comparer les qualités physiques mesurées à celles calculées théoriquement sur la base des données entrées par l'utilisateur 7 (représentées par des cercles sur la figure 4).

Grâce à cette comparaison, il est possible d'orienter les sollicitations des qualités physiques 200 soit en s'orientant sur les faiblesses de l'utilisateur (objectif de réduire l'écart entre les cercles et les étoiles de la figure 4), soit en sollicitant de préférence les forces de l'utilisateur (objectif de centrer la préparation sur les qualités pour lesquelles les étoiles sont de niveau plus élevé que les cercles). En d'autres mots la première association des qualités physiques 200 à des niveaux 100 en se basant seulement sur les données entrées par l'utilisateur 7 n'est pas statique, mais dynamique et prend en considération les niveaux des qualités physiques mesurés pendant l'exécution des tests physiques (ou exercices-repère) initiaux.

Dans une autre variante non seulement les niveaux théoriques de chaque qualité physique sont modifiés, mais le type d'exercices initiaux proposés à l'utilisateur peut être également modifié.

Le système est donc évolutif : en d'autres mots, le placement à priori du niveau de chaque qualité physique de l'utilisateur (les cercles) n'est pas fixe, mais change dans le temps car le système selon l'invention est évolutif en fonction des niveaux mesurés pour les utilisateurs précédents et/ou pour le même utilisateur.

Le test initial est également évolutif dans le temps, en fonction des paramètres mesurés par des utilisateurs précédents pendant le test initial.

Dans une variante préférentielle l'utilisateur lors de l'entrée de ses données personnelles peut également indiquer la qualité physique 200 qu'il préfère ou aime améliorer : dans ce cas le pourcentage (ou poids relatif) de cette qualité physique sera augmenté en conséquence.

Après que l'utilisateur 7 a exécuté les exercices initiaux, des exercices sont sélectionnés automatiquement dans l'espace illustré sur la figure 3C, en fonction des objectifs sélectionnés par l'utilisateur 7 (par exemple du sport pour lequel il s'entraîne, ou de la partie du corps qu'il souhaite rééduquer), et d'une ou plusieurs qualités physiques 200 à renforcer et/ou activer déterminées en fonction du niveau 100 de l'utilisateur 7 pour cette qualité.

Avantageusement le dispositif mobile 5 comprend des moyens pour entrer une commande haptique et/ou vocale de l'utilisateur lors de l'exécution d'un exercice, cette commande indiquant la sensation de l'utilisateur 7 pendant cet exercice. En d'autres termes cette commande constitue un feedback qui est pris également en compte dans le système selon l'invention pour la sélection des exercices proposés à l'utilisateur 7.

Dans une autre variante, l'accéléromètre 3, et non pas le dispositif mobile 5, comprend ces moyens pour entrer une commande haptique et/ou vocale de l'utilisateur 7.

Dans le contexte de cette invention l'expression « moyen de commande haptique » indique tout organe ou moyen qui performe une fonction s'il est touché par un utilisateur ou par un moyen tel qu'un stylet. Des exemples d'organes de commande haptiques comprennent des boutons-poussoirs ou un écran tactile. Les moyens pour entrer une commande vocale peuvent comprendre un microphone.

Dans une variante préférentielle ces moyens comprennent deux moyens de commande haptiques, par exemple deux boutons, un bouton permettant d'entrer une commande correspondant à une sensation positive (« J'aime », « I feel good »), et un bouton permettant d'entrer une commande correspondant à une sensation négative (« Je n'aime pas »).

Dans une autre variante un seul bouton permettant d'entrer une commande correspondant à une sensation positive est présent.

Dans une autre variante le nombre de moyens de commande haptiques est supérieur à deux, afin de considérer les nuances intermédiaires entre les sensations « j'aime » et « je n'aime pas ».

Dans une autre variante le dispositif mobile 5 et/ou l'accéléromètre 3 comprennent des moyens d'insertion d'une commande vocale de la part de l'utilisateur : des expressions telles que « J'aime », « I like it », etc. peuvent donc être reconnues et utilisées par le système selon l'invention en tant que feedback de l'utilisateur 7 pour sélectionner automatiquement des exercices. Dans une autre variante la commande est détectée par l'accéléromètre 3 sur la base d'impulsions par lui repérées : par exemple l'utilisateur peut directement taper sur l'accéléromètre 3 un nombre entier de fois, par exemple deux fois, pour indiquer qu'il aime l'exercice qu'il est train d'exécuter. Dans une variante préférentielle il tape deux fois de manière rapide, par exemple les deux impulsions étant séparées d'une plage temporelle inférieure à 1 seconde, par exemple inférieure à 0.5 secondes, pour indiquer qu'il aime l'exercice.

L'utilisation du feedback de l'utilisateur permet de tenir compte de la réaction non seulement physique, mais aussi émotionnelle de l'utilisateur 7 exécutant des exercices, qui est utilisée pour choisir les exercices à lui proposer.

Par exemple le système selon l'invention peut mesurer que pendant une course à pied l'utilisateur utilise les jambes de façon asymétrique : un exercice peut alors être proposé pour essayer de corriger cette asymétrie. Si cependant l'utilisateur aime courir de cette façon asymétrique, il peut donner la commande « J'aime » au système qui donc n'essaie plus de corriger l'asymétrie, car l'utilisateur est à l'aise en courant ainsi.

En d'autres mots le système selon l'invention permet d'arriver à l'objectif de l'entraînement ou de l'activation, ou du plaisir ou encore de la réhabilitation en tenant compte de la façon dans laquelle l'utilisateur 7 se sent à l'aise, même si cette façon n'est pas la plus efficace d'un point de vue théorique.

Dans une autre variante le dispositif mobile 5 et/ou l'accéléromètre 3 permettent de déterminer la signature du mouvement de l'utilisateur sur la base de données d'accélérations captées par l'accéléromètre 3.

Dans le contexte de cette invention, l'expression « signature du mouvement » indique comment les valeurs d'accélérations mesurées varient dans le temps. Elle indique également comment des paramètres calculés sur la base des accélérations, ou une combinaison de ces paramètres, varient dans le temps. Des exemples de ces paramètres, simples ou complexes, sont : force, puissance, vitesse, stabilité, temps de réaction, temps de vol, temps de contact, raideur, réactivité, asymétrie, inclinaison, fatigue, risque de blessure.

Donc le système selon l'invention proposera à deux utilisateurs 7 ayant les mêmes objectifs mais des signatures de mouvements différentes des exercices différents.

Avantageusement la base de données des exercices proposés à l'utilisateur est donc évolutive, c'est-à-dire qu'elle n'est pas statique, mais change dans le temps.

Pendant l'exécution de ces exercices, l'accéléromètre 3 mesure des accélérations qui sont transmises au dispositif mobile 5, dont le processeur 23 est arrangé pour calculer, sur la base des données d'accélérations, des paramètres musculaires. Dans une autre variante l'accéléromètre 3 comprend un processeur permettant de calculer directement ces paramètres musculaires.

Dans une variante préférentielle ces paramètres musculaires comprennent :
- la force
- la puissance
- la vitesse
- la stabilité
- le temps de réaction
- le temps de vol et le temps de contact au sol
- la raideur
- la réactivité
- l'asymétrie
- la répétabilité
- le nombre de répétition
- les inclinations/les angles
- la réaction
- le rythme
- la hauteur
- l'équilibre
- l'oscillation
- toute combinaison de deux ou plusieurs paramètres ci-dessus.

Les documents EP2582295 et EP2582294, déposés par la demanderesse et également incorporés par référence, décrivent des exemples de paramètres musculaires pouvant être calculés par le système selon l'invention.

Selon un aspect indépendant de l'invention le système selon l'invention permet une détermination automatique par le dispositif mobile 5 et/ou par l'accéléromètre 3 des objectifs de l'utilisateur. En d'autres mots dans les systèmes connus, l'utilisateur 7, seul et/ou à l'aide de son coach, déterminait les objectifs de son entrainement et/ou réhabilitation. Dans une variante de la présente invention, le dispositif mobile 5 et/ou l'accéléromètre 3 sont en mesure de déterminer cet objectif, sur la base :
- des données personnelles entrées par l'utilisateur 7 (âge, sexe, etc.) ;
- des mesures réalisées par l'accéléromètre ;
- de l'objectif utilisateur choisi ;
- des feedbacks de l'utilisateur (ses sensations pendant l'exécution de l'exercice et/ou la signature de son mouvement).

Dans une variante préférentielle la détermination automatique des objectifs est également évolutive pendant l'exécution des exercices par l'utilisateur 7.

Dans une variante préférentielle les exercices proposés à l'utilisateur 7 constituent une unité d'entraînement, qui a une durée de quelques minutes, par exemple de 10 minutes. Dans le contexte de cette invention, le mot « séance » indique alors une unité d'entraînement, ou un assemblage de plusieurs unités d'entraînement. Une séance peut donc avoir une durée totale d'un nombre entier de semaines, par exemple de 3 semaines. Dans une variante des exercices-repère sont placés dans les séances de façon à faire évoluer l'entraînement selon le niveau atteint au temps t+1 comparativement au niveau atteint au temps t. Dans une autre variante chaque séance est suivie par un test d'évaluation.

Dans une variante les séances sont composées automatiquement de manière à respecter les règles suivantes :
- si les résultats des tests sont inférieurs aux attentes pour une qualité physique donnée, des exercices respectant les résultats des tests sont proposés à l'utilisateur 7, par exemple des exercices de niveau inférieur;
- le niveau de difficulté des exercices sélectionnés s'évertuera à ne pas être supérieur d'un point au niveau testé ;
- le poids relatif de chaque qualité physique sollicitée par l'ensemble d'exercices d'une séance respecte le marquage de l'objectif utilisateur et/ou l'objectif utilisateur et/ou les paramètres de pondération (sensations, signature du geste...).

En résumant il est donc possible de classer et sélectionner des exercices sur la base du niveau de l'exercice et/ou de l'utilisateur, des qualités physiques à améliorer, de l'objectif utilisateur sélectionné, de la sensation de l'utilisateur pendant l'exécution de l'exercice, mais aussi sur la base de la signature du mouvement de l'utilisateur.

## Revendications

1. Procédé de sélection automatique d'exercices physiques (300) à proposer à un utilisateur (7) via un système de sélection automatique d'exercices physiques (300) à proposer audit utilisateur (7), ledit système comprenant :
- un capteur de mouvement, par exemple un accéléromètre, (3) arrangé pour être porté par ledit utilisateur (7)
- une base de données dans laquelle une collection prédéfinie d'exercices physiques est classifiée en fonction à la fois d'objectifs utilisateurs, de qualités physiques (200) que ces exercices physiques permettent de renforcer et/ou activer, et pour chaque qualité physique (200) de niveaux (100) d'utilisateurs (7) pour lesquels ces exercices physiques sont appropriés, un objectif utilisateur étant un objectif de l'utilisateur (7) pour lequel il va exécuter des exercices physiques,
le système étant configuré pour permettre à l'utilisateur :
- d'introduire des données personnelles comprenant au moins un objectif utilisateur et
- d'introduire un feedback de l'utilisateur indiquant une sensation de l'utilisateur pendant l'exécution de l'exercice physique;
le procédé comprenant les étapes suivantes :
- introduction par l'utilisateur (7) de données personnelles dans ledit capteur de mouvement, lesdites données personnelles comprenant au moins un objectif utilisateur ;
- test à l'aide dudit capteur de mouvement (3) du niveau de l'utilisateur (7) pour différentes qualités physiques (200) ;
- sélection automatique par ledit système d'au moins un exercice physique (300) dans ladite base de données sur la base dudit objectif utilisateur introduit par l'utilisateur (7), d'une ou plusieurs qualités physiques (200) à renforcer et/ou activer déterminées en fonction du niveau (100) de l'utilisateur (7) pour cette qualité, et d'au moins un feedback de l'utilisateur (7), ledit feedback indiquant la sensation de l'utilisateur (7) pendant l'exécution de l'exercice physique (300).

2. Le procédé selon la revendication 1, ledit feedback incluant une commande haptique ou vocale introduite volontairement par l'utilisateur (7) pendant l'exécution de l'exercice physique afin d'indiquer une sensation.

3. Le procédé selon l'une des revendications 1 ou 2, ledit feedback incluant une signature du mouvement de l'utilisateur (7) déterminée automatiquement sur la base de données d'accélérations captées par ledit capteur de mouvement (3).

4. Le procédé selon l'une des revendications 1 à 3, les qualités physiques (200) comprenant au moins deux des qualités physiques suivantes : core stability, flexibilité, endurance, puissance, agilité, perception.

5. Le procédé selon l'une des revendications 1 à 4, les niveaux (100) étant au moins deux, et étant indicatifs de façon progressive des qualités physiques dudit utilisateur et/ou de la difficulté de chaque exercice physique.

6. Le procédé selon l'une des revendications 1 à 5, lesdits objectifs utilisateurs incluant un sport que l'utilisateur souhaite entraîner et/ou une pathologie.

7. Le procédé selon l'une des revendications 3 à 6, ladite sélection automatique étant réalisée en fonction de ladite signature du mouvement dudit utilisateur (7) pendant l'exécution de l'exercice physique (300).

8. Le procédé selon l'une des revendications 1 à 7, comprenant la mesure du niveau (100) de l'utilisateur (7) pour au moins un paramètre physique (200) et/ou une pathologie au cours de l'exécution des exercices physiques sélectionnés, et une ultérieure sélection de nouveaux exercices physiques en fonction de ce niveau, des objectifs de l'utilisateur et du feeling de l'utilisateur.

9. Le procédé selon l'une des revendications 3 à 8, ladite signature du mouvement dudit utilisateur (7) comprenant l'évolution temporelle d'au moins un des paramètres suivants et/ou une combinaison d'au moins deux des paramètres suivants : force, puissance, vitesse, stabilité, temps de réaction, temps de vol, temps de contact, raideur, réactivité, asymétrie, inclinaison, fatigue, risque de blessure, nombre de répétition, réaction, rythme, hauteur, équilibre, oscillation.

10. Le procédé selon l'une des revendications 1 à 9, lesdites données personnelles comprenant au moins une des données suivantes : âge, poids, taille, sexe de l'utilisateur, pathologie.

11. Le procédé selon l'une des revendications 1 à 10, comprenant l'évolution et modification automatique dudit test en fonction des différentes qualités physiques (200) mesurées pendant l'exécution des exercices physiques (300).

12. Le procédé selon l'une des revendications 1 à 11, un ou plusieurs exercices physiques (300) étant regroupés en unité d'entraînement, une ou plusieurs unités d'entraînement étant regroupées en séances ayant une durée totale d'un nombre entier de semaines, par exemple de 3 semaines, des exercices-repères étant placés dans chaque séance de façon à faire évoluer l'entraînement selon le niveau (100) atteint au temps t+1 comparativement au niveau (100) atteint au temps t.

13. Système de sélection automatique d'exercices physiques (300) à proposer à un utilisateur (7), comprenant :
- un capteur de mouvement, par exemple un accéléromètre, (3) arrangé pour être porté par ledit utilisateur (7)
- une base de données dans laquelle une collection prédéfinie d'exercices physiques est classifiée en fonction à la fois d'objectifs utilisateurs, de qualités physiques (200) que ces exercices physiques permettent de renforcer et/ou activer, et pour chaque qualité physique (200) de niveaux (100) d'utilisateurs (7) pour lesquels ces exercices physiques sont appropriés
- le système étant configuré pour permettre à l'utilisateur d'introduire de données personnelles comprenant au moins un objectif utilisateur et un feedback de l'utilisateur indiquant une sensation de l'utilisateur pendant l'exécution d'un exercice physique; et de sélectionner automatiquement au moins un exercice physique dans ladite base des données sur la base dudit objectif utilisateur introduit par l'utilisateur (7), d'une ou plusieurs qualités physiques (200) à renforcer et/ou activer déterminées en fonction du niveau (100) de l'utilisateur pour cette qualité, et d'au moins un feedback de l'utilisateur (7), ledit feedback indiquant la sensation de l'utilisateur (7) pendant l'exécution de l'exercice physique.

## Patentansprüche

1. Verfahren zur automatischen Auswahl von körperlichen Übungen (300), die einem Nutzer (7) über ein System zur automatischen Auswahl von körperlichen Übungen (300), die dem Nutzer (7) vorgeschlagen werden sollen, angeboten werden sollen, wobei das System umfasst:
- ein Bewegungssensor, z. B. einen Beschleunigungsmesser, (3), der so angeordnet ist, dass er von dem Nutzer (7) getragen wird
- eine Datenbank, in der eine vordefinierte Sammlung von körperlichen Übungen sowohl nach Nutzerzielen als auch nach körperlichen Fähigkeiten (200), die durch diese körperlichen Übungen gestärkt und/oder aktiviert werden können, und für jede körperliche Fähigkeit (200) nach Leistungsstufen (100) von Nutzern (7), für die diese körperlichen Übungen geeignet sind, klassifiziert wird, wobei ein Nutzerziel ein Ziel des Nutzers (7) ist, für das er die körperlichen Übungen ausführen wird,
wobei das System so konfiguriert ist, dass es dem Nutzer ermöglicht:
- persönliche Daten einzugeben, die mindestens ein Nutzerziel umfassen, und
- ein Nutzerfeedback einzugeben, das eine Empfindung des Nutzers während der Ausführung der körperlichen Übungen angibt;
wobei das Verfahren die folgenden Schritte umfasst:
- Eingeben von persönlichen Daten durch den Nutzer (7) in den Bewegungssensor, wobei die persönlichen Daten mindestens ein Nutzerziel umfassen;
- Messen des Niveaus des Nutzers (7) für verschiedene körperliche Fähigkeiten (200) mit dem Bewegungssensor (3);
- automatisiertes Auswählen mindestens einer körperlichen Übung (300) aus der Datenbank durch das System auf der Grundlage des vom Nutzer (7) eingegebenen Nutzerziels, einer oder mehrerer zu stärkender und/oder zu aktivierender körperlicher Fähigkeiten (200), die in Abhängigkeit vom Niveau (100) des Nutzers (7) für diese Fähigkeit bestimmt werden, und mindestens eines Feedbacks des Nutzers (7), wobei das Feedback die Empfindung des Nutzers (7) während der Ausführung der körperlichen Übung (300) angibt.

2. Das Verfahren nach Anspruch 1, wobei das Feedback eine haptische oder sprachliche Steuerung einschließt, die vom Nutzer (7) während der Ausführung der körperlichen Übung absichtlich eingegeben wird, um eine Empfindung anzuzeigen.

3. Das Verfahren nach einem der Ansprüche 1 oder 2, wobei das Feedback eine Bewegungssignatur des Nutzers (7) einschließt, die automatisch auf der Grundlage von Beschleunigungsdaten bestimmt wird, die von dem Bewegungssensor (3) erfasst werden.

4. Das Verfahren nach einem der Ansprüche 1 bis 3, wobei die körperlichen Fähigkeiten (200) mindestens zwei der folgenden körperlichen Fähigkeiten umfasst: Rumpfstabilität, Gelenkigkeit, Ausdauer, Kraft, Beweglichkeit, Wahrnehmungsvermögen.

5. Das Verfahren nach einem der Ansprüche 1 bis 4, wobei die Stufen (100) mindestens zwei umfassen und in progressiver Weise die körperlichen Fähigkeiten des Nutzers und/oder den Schwierigkeitsgrad der jeweiligen körperlichen Übung anzeigen.

6. Das Verfahren nach einem der Ansprüche 1 bis 5, wobei die Nutzerziele eine Sportart, die der Nutzer trainieren möchte, und/oder eine Erkrankung umfassen.

7. Das Verfahren nach einem der Ansprüche 3 bis 6, wobei die automatische Auswahl auf der Grundlage der Bewegungssignatur des Nutzers (7) während der Ausführung der körperlichen Übung (300) erfolgt.

8. Das Verfahren nach einem der Ansprüche 1 bis 7, umfassend das Messen des Niveaus (100) des Nutzers (7) für mindestens einen körperlichen Parameter (200) und/oder eine Erkrankung während der Ausführung der ausgewählten körperlichen Übungen und eine anschließende Auswahl neuer körperlicher Übungen auf der Grundlage dieses Niveaus, der Ziele und der Empfindung des Nutzers.

9. Das Verfahren nach einem der Ansprüche 3 bis 8, wobei die Bewegungssignatur des Nutzers (7) die zeitliche Entwicklung von mindestens einem der folgenden Parameter und/oder eine Kombination von mindestens zwei der folgenden Parameter umfasst: Kraft, Leistung, Geschwindigkeit, Stabilität, Reaktionszeit, Flugdauer, Kontaktzeit, Steifheit, Reaktionsfähigkeit, Asymmetrie, Neigung, Ermüdung, Verletzungsrisiko, Anzahl der Wiederholungen, Reaktionsvermögen, Rhythmus, Höhe, Gleichgewicht, Schwankung.

10. Das Verfahren nach einem der Ansprüche 1 bis 9, wobei die persönlichen Daten mindestens eine der folgenden Angaben umfassen: Alter, Gewicht, Größe, Geschlecht des Nutzers, Erkrankung.

11. Das Verfahren nach einem der Ansprüche 1 bis 10, umfassend die automatische Weiterentwicklung und Modifikation der Messung in Abhängigkeit von den verschiedenen körperlichen Fähigkeiten (200), die während der Ausführung der körperlichen Übungen (300) gemessen werden.

12. Das Verfahren nach einem der Ansprüche 1 bis 11, wobei eine oder mehrere körperliche Übungen (300) in einer Trainingseinheit zusammengefasst werden, wobei eine oder mehrere Trainingseinheiten in Einheiten mit einer Gesamtdauer von einer ganzzahligen Anzahl von Wochen, beispielsweise 3 Wochen, zusammengefasst werden, wobei Referenzübungen in jede Sitzung integriert werden, um zu gewährleisten, dass sich das Training entsprechend dem zum Zeitpunkt t+1 erreichten Niveau (100) im Vergleich zu dem zum Zeitpunkt t erreichten Niveau (100) verbessert.

13. System zur automatischen Auswahl von körperlichen Übungen (300), die einem Nutzer (7) vorgeschlagen werden sollen, umfassend:
- einen Bewegungssensor, z. B. einen Beschleunigungsmesser, (3), der so angeordnet ist, dass er von dem Nutzer (7) getragen
wird
- eine Datenbank, in der eine vordefinierte Sammlung von körperlichen Übungen sowohl nach Nutzerzielen als auch nach körperlichen Fähigkeiten (200), die durch diese körperlichen Übungen gestärkt und/oder aktiviert werden können,
klassifiziert wird, und für jede körperliche Fähigkeit (200) von Leistungsstufen (100) von Nutzern (7), für die diese körperlichen Übungen geeignet sind wobei
das System derart konfiguriert ist, dass es dem Nutzer ermöglicht, persönliche Daten einzugeben, die mindestens ein Nutzerziel und ein Nutzerfeedback umfassen, das eine Empfindung des Nutzers während der Ausführung einer körperlichen Übung angibt, und automatisch mindestens eine körperliche Übung aus der Datenbank auf der Grundlage des vom Nutzer eingegebenen Nutzerziels (7), einer oder mehrerer zu stärkender und/oder zu aktivierender körperlicher Fähigkeiten (200), die in Abhängigkeit vom Niveau (100) des Nutzers für diese Fähigkeit bestimmt werden, und mindestens eines Feedbacks des Nutzers (7) auszuwählen, wobei das Feedback die Empfindung des Nutzers (7) während der Ausführung der körperlichen Übung angibt.

## Claims

1. Method for automatically selecting physical exercises (300) to propose to a user (7), via a system for automatically selecting physical exercises (300) to be offered to said user (7), said system comprising :
- a movement sensor, for example an accelerometer, (3) arranged to be worn by said user (7),
- a database in which a predefined collection of physical exercises is classified according simultaneously to user objectives, to physical qualities (200) that these physical exercises make it possible to strengthen and/or activate, and for each physical quality (200), to levels (100) of users (7) for which these physical exercises are appropriate, a user objective being an objective of the user (7) for which he will perform physical exercises,
the system being configured for allowing the user to:
- enter by the user (7) personal data in said movement sensor, said personal data comprising at least a user objective, and
- enter a feedback of the user indicating a sensation of the user during the execution of the physical exercise;
the method comprising the following steps:
- enter by the user (7) personal data in said movement sensor, said personal data comprising at least a user objective;
- test by means of said motion sensor (3) of the level of the user (7) for different physical qualities (200);
- automatically select by said system at least one physical exercise (300) in said database on the basis of said user objective entered by the user (7), one or several physical qualities to be reinforced and/or activated as determined according to the level (100) of the user (7) for this quality, and at least one user feedback of the user (7), said feedback indicating the sensation of the user (7) during the performance of said physical exercise (300).

2. The method according to claim 1, wherein said feedback includes a haptic or vocal command deliberately entered by the user (7) during the performance of the physical exercise in order to indicate a sensation.

3. The method according to one of claims 1 or 2, wherein said feedback includes a signature of the movement of the user (7) determined automatically on the basis of acceleration data collected by said movement sensor (3).

4. The method according to one of claims 1 to 3, wherein the physical qualities (200) comprise at least two of the following physical qualities: core stability, flexibility, endurance, power, agility, perception.

5. The method according to one of claims 1 to 4, wherein the levels (100) are at least two and indicate in a progressive manner the physical qualities of said user and/or the difficulty of each physical exercise.

6. The method according to one of claims 1 to 5, wherein said user objectives include a sport that the user wishes to train and/or a pathology.

7. The method according to one of claims 3 to 6, wherein said automatic selection is effected on the basis of said signature of the movement of said user (7) during the performance of the physical exercise (300).

8. The method according to one of claims 1 to 7, comprising the measuring of the level (100) of the user (7) for at least one physical parameter (200) and/or a pathology during the performance of the selected physical exercises, and a subsequent selection of new physical exercises according to this level, of the user objectives and of the user's feeling.

9. The method according to one of claims 3 to 8, wherein said signature of the movement of said user (7) comprises the evolution over time of at least one of the following parameters and/or a combination of at least two of the following parameters: force, power, speed, stability, reaction time, flight time, contact time, stiffness, responsiveness, asymmetry, inclination, fatigue, risk of injury, number of repetitions, reaction, rhythm, height, balance, oscillation.

10. The method according to one of claims 1 to 9, wherein said personal data comprise at least one of the following data: age, weight, height, gender of the user, pathology.

11. The method according to one of claims 1 to 10, comprising the evolution and automatic modification of said test according to the different physical qualities (200) measured during the execution of the physical exercises (300).

12. The method according to one of claims 1 to 11, wherein one or several physical exercises (300) are regrouped in a training unit, wherein one or several training units are regrouped in sessions having a total duration of a whole number of weeks, for example 3 weeks, wherein reference exercises are integrated in each session so as to cause the training to evolve according to the level (100) achieved at the time t+1 as compared to the level (100) reached at the time t.

13. System for automatically selecting physical exercises (300) to propose to a user (7), comprising:
- a motion sensor, for example an accelerometer (3), designed for being worn by said user (7),
- a database in which a predefined collection of physical exercises is classified according simultaneously to user's objectives, to physical qualities (200) that these physical exercises allow to reinforce and/or activate, and for each physical quality (200) to the levels (100) of users (7) for which these physical exercises are appropriate,
- wherein the system is configured to allow the user to input personal data comprising at least one user objective and a feedback of the user indicating a sensation of the user during the execution of a physical exercise; and to automatically select at least one physical exercise in said database on the basis of said user objective entered by the user (7), of one or several physical qualities (200) to be reinforced and/or activated as determined according to the user's level (100) for this quality, and at least one feedback from the user (7), said feedback indicating the sensation of the user (7) during the performance of the physical exercise.
